# EUROPEAN PATENT SPECIFICATION

(11) **EP 2 849 654 B1**
(45) Date of publication and mention of the grant of the patent: **20.06.2018**
(21) Application number: 13787775.9
(22) Date of filing: 10.05.2013
(51) Int. Cl.: A61C 8/00, A61C 8/02, A61F 2/28

(54) **DENTAL DEVICES FOR EXTRACTION SITE RECONSTRUCTION**
ZAHNÄRZTLICHE VORRICHTUNGEN ZUR REKONSTRUKTION EINER EXTRAKTIONSSTELLE
DISPOSITIFS DENTAIRES POUR RECONSTRUIRE UNE ALVÉOLE D'EXTRACTION

(30) Priority: 10.05.2012 US 201261645556 P
(43) Date of publication of application: 25.03.2015
(73) Proprietor: Zadeh, Homayoun H., Woodland Hills, CA 91367 (US)
(72) Inventor: Zadeh, Homayoun H., Woodland Hills, CA 91367 (US)
(74) Representative: Kremer, Simon Mark
(86) International application number: PCT/US2013/040665
(87) International publication number: WO 2013/170224

(56) References cited:
- DE-A1-102005 039 382
- US-A- 3 979 828
- US-A- 4 186 486
- US-A- 4 531 916
- US-A- 5 511 565
- US-A1- 2010 331 997
- US-B1- 6 394 806

## Description

### Field of the Art

The present invention is dental devices for preservation and/or reconstruction of extraction sockets. Specifically, caps protect and seal the extraction socket and bone graft material placed within the sockets. Cages (not claimed) provide structural support following tooth extraction when portions of the extraction socket wall are pathologically destroyed.

### Background

Dental extraction of teeth or dental implants is necessitated by pathological diseases or trauma that damages the bone and tissue surrounding the socket. When the tissue or bone structure of the socket is compromised, the prognosis for successful repair is diminished. Extraction of teeth or implants initiates a cascade of healing responses that typically accompany growth of tissues to quickly fill and cover the resulting socket defects. The sequence of healing initially entails filling of the socket with a blood clot, the blood clot is replaced in turn by granulation tissue, followed by deposition of woven bone, and eventually maturing to lamellar bone. During the early steps in this healing process, the extraction socket has an opening exposed to the oral cavity and particularly during the stage when the blood clot fills the defect followed by granulation, a time period exists when oral microorganisms and food can contaminate the healing process within the socket. In some instances, the process may result in pathologic healing conditions such as dry socket, which is accompanied by painful symptoms, delayed healing, and necrosis of significant bone within the socket.

Even under best circumstances, the healing of extraction sockets is usually accompanied by substantial loss of alveolar bone, which is more pronounced on the facial (buccal or labial) aspects of the socket. The reduced alveolar bone volume will result in a esthetic and functional deficits, even when lost teeth are replaced by dental prostheses, such as implant-supported, tooth-supported or mucosal supported prostheses. Aside from the aesthetic deficit, the reduced alveolar housing may lead to speech and/or masticatory deficits. Alternatively, complex augmentation procedures may be required to correct the alveolar ridge defects resulting from resorption of extraction sockets and the associated morbidity and cost. Similar processes occur within failed implant sites, where the failed implants have been removed.

Under existing practice, the dentist or surgeon has two basic options. The first option is to allow the extraction socket to heal naturally. As outlined above, this approach may lead to substantial bone and soft tissue loss. The second option is to augment the extraction socket by placement of biomaterial within the socket. Whether the socket is filled with biomaterial or is left unfilled, a major concern is exposure of the socket or the graft to the oral environment. Attempts to seal the socket opening usually entail placement of a resorbable or non-resorbable barrier membrane at the opening. This usually involves reflection of a surgical flap to place the membrane between bone and the gingival margin.

However, the manual placement of a synthetic membrane often produces unsatisfactory results because 1) the placement of the membrane frequently requires additional surgical intervention and manipulation of the soft tissue to place the membrane below gingival margins; 2) the use of the membrane can cause collapse of the repaired bone and overlying soft tissue; 3) the clinician frequently encounters technical difficulty in attaching a flat membrane to the complex topography of an extraction socket); 4) the premature exposure of the healing bone and tissue in the extraction site can lead to the development of infection that compromises healing, or 5) the socket has the tendency to heal and regenerate bone and tissue in unpredictable geometries that may impede future treatment or require additional surgical intervention. Thus, the existing membrane materials and membrane placement materials do not always work well for their intended purpose.

US Patent No 3,979,828 describes a dental prosthetic device and prosthetic dentistry method. The device includes an implant disposable in a fresh extraction site or a surgically produced extraction site, and a crown removably attachable to the implant.

US Patent Application US 2010/0331997 describes an implant intended for introduction into an alveolar space. The implant is configured as an absorbable composite body made of at least a first and second part body.

US Patent No 4,531,916 describes a device implant with expanded gingival interface. The device has a root structure, a cervical segment and a gingival interface.

US Patent No 4,186,486 describes a prosthetic device particularly useful for artificial dental implants, which includes a portion such as a tooth root adapted for mounting to a bone tissue.

US Patent No 6,394,806 describes a healing cap for covering of an implant installed in a patient's mouth. The cap comprises a proximal end and distal end.

Dental socket caps comprising attachment means in the form of grooves operatively connected to a suture passage traversing a top portion of the socket cap are not described in the above-cited prior art.

Accordingly, a need exists for devices and procedures that are specially designed to protect the integrity and geometry at the extraction site and to preserve the integrity of bone and soft tissue during the healing period while enhancing the surgeon's ability to prepare and preserve the site for healing and further treatment. There is also a need for devices and procedures that enhance the post-healing structure of the site, reduce the potential for infection, and that more completely seal the site during healing.

### Summary of Invention

The present invention is defined by the appended independent claim 1. Preferred embodiments of the invention are defined in the dependent claims.

Disclosed are surgical devices, systems, and methods for maintaining the integrity of an the alveolar ridge following tooth or implant extraction or other dental or maxillofacial surgery. Specifically, the devices are dental extraction socket caps and socket cages that seal an extraction site and provide support for repair, regrowth, or surgical intervention at the site, including the surrounding bone and soft tissue, especially where part of the socket structure is damaged or destroyed. These devices are suitable for any surgical procedure that creates an extraction site where the healing of bone and soft tissue is contemplated such that the integrity of the bone and soft tissue, the quality and geometry at the revascularization of the site, and the microbiological environment is desired to be controlled. The devices and methods of the invention apply to enhance general healing at the site and improve the bone and tissue to prepare for future placement of a prosthetic device.

The devices are pre-formed, although a set of devices with specific sizes may be offered to accommodate the individual clinical situations presented by different patients. In some embodiments, the size and configuration of the devices is modified by design or by the clinician using available materials, e.g., either through construction of the devices from thermoplastic or by manipulating elements at the device formed from a material that is moldable. In other embodiments, a series of size-customized devices are constructed for selection according to each patent site using three-dimensional printing, rapid prototyping or other manufacturing processes. Because the devices have structural integrity that extends into the interior of extraction sockets, they offer a simpler method for sealing the socket and maintaining the space to support proper healing than existing membranes that require customized surgical intervention to merely cover the extraction site. Because the devices are solid and pre-formed, placement is simpler, less expensive, and preserves time and materials while creating a more uniform final result at the healed extraction site. One of the most common complications of current methods of ridge preservation with membranes is exposure of graft material because of loss of integrity of membranes. Due to their structural integrity and design features that are complementary to the shape of common extraction sockets, the socket devices are more likely to protect the graft material during their the healing period.

The devices are fabricated from known biocompatible materials that promote the aseptic healing of surgical sites, both those involved in dental surgery and in general surgical applications, and are antiseptically sealed and prepared for use during manufacturer or, at the clinicians' option, in the surgeon's office. Because each structure at a predetermined size and configuration is uniform in design as the result of prefabrication, the surgical procedures for placing and securing the devices at the extraction site are standardized and advantageous over existing procedures where customized membrane materials are used. Moreover, current membranes are flat and do not conform well to the complex anatomy of extraction sockets. The extraction socket devices have been fabricated based on common anatomical structure and dimensions of extraction socket of various teeth.

The present invention includes the devices disclosed here and their uses in the surgical procedures required for placement of the devices as described below. These procedures include the specific differences and advantages in surgical preparation at the site, the placement of the devices, the preparation of the extraction site, the insertion and securing the devices at the site (including forming a competent seal to the tissue), and the removal of the devices upon completed healing of bone and soft tissue. The attendant surgical procedures may include harvesting of bone from donor sites, tissue grafting, or modeling of bone or soft tissue and the general preparation of the extraction site for the future placement and removal of prosthetic devices.

### Description of the Figures

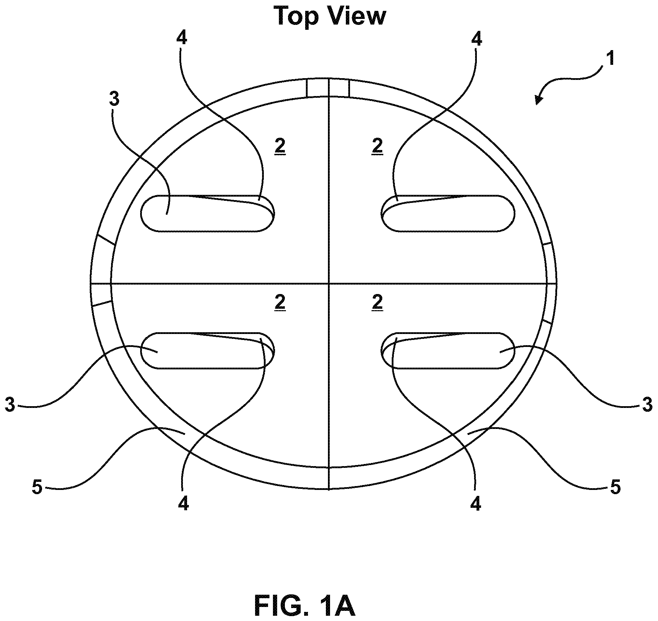

Figures 1A - 1E are top, diagonal, interproximal, facial/buccal/lingual, and bottom views of a socket cap having a dome portion and a downward projection that occupies at least a portion of the internal volume of an extraction site.

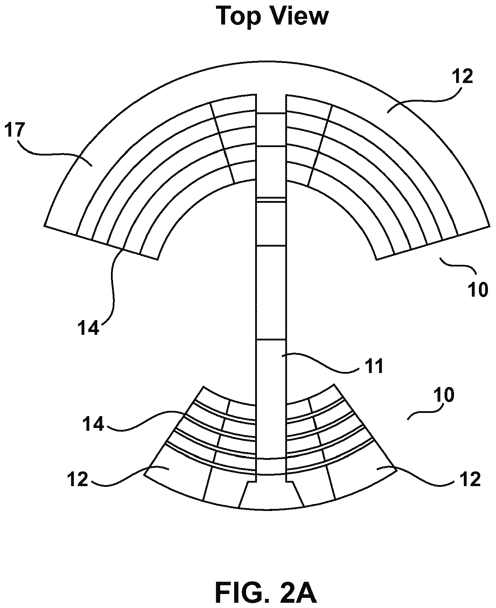

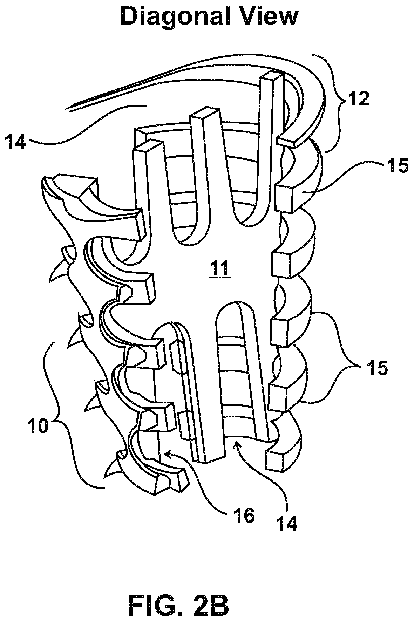

Figure 2A-2E are top, diagonal, side, facial/buccal and lingual/palatal view of a socket cage or socket scaffold (not claimed) having perforated walls. The walls facing facial/buccal and lingual/palatal aspects of extraction socket are curved and have a tapered dimension formed from a series of spaced apart rib-like projections that act as braces and stabilizers for the surrounding tissue.

### Detailed Description of Invention

The first embodiment of the invention is an extraction socket cap that is shaped and sized to seal an extraction site about the periphery thereof during a healing period. The shape of the socket cap can be rounded square, rounded triangle, circular, oval, or any desired shape depending on the anatomic characteristics of the extraction sites. The design incorporates features to allow the surgeon to advantageously place and position the socket cap on the extraction site while insuring that proper positioning and effective sealing about the gingival margin is secure prior to affixing the cap by suture attachment, adhesives, or fixation screws to the surrounding soft tissue.

The general dimensions and orientation of the structures shown in Figures 1A through F are examples of different embodiments of the invention. The socket cap features a top or cap portion designed to seal about the periphery of an extraction site. The top portion is mated to a bottom portion that extends downward and away from the cap. The bottom (intaglio) portion may have perforations formed a length thereof to form openings therein to facilitate the exchange of fluid, cells and materials between the interior of the extraction site and the surrounding tissues. Alternatively, the lower portion may be fluid sealed to prevent fluid transport across the extraction site above or below the original margin. The solid bottom portion of the cap will prevent influx of microorganisms from the oral cavity and provide an improved seal at the opening of the extraction site. The downward extensions are formed to project on all sides of the socket cap, although formation along at least two sides of the overall structure of thereof is preferred. The number of the perforations is not critical but may be varied based on the ease of manufacturing or a process of removing the material to form the perforations while maintaining the strength of the downward extensions of the perforations along their length. The perforations are typically formed along substantially the entire length thereof, with the exception of the superior or upper-most aspects of a panel forming the lower portion where joins the dome-shaped part of the upper portion or cap.

The bottom portion of the cap may also be comprised of a series of panels formed into a unitary structure that provides an intact housing extending downward and generally perpendicular from the plane formed by the outer edge of the top portion. In this configuration, the attachment and seal between the top portion and the bottom portion effectively form a sealing enclosure for the internal volume of the extraction site separating the volume therein from the remainder of the oral cavity. Moreover, the bottom portion may be sealed about the bottom circumference and may have a sealed intaglio surface that prevents any fluid or material from entering the housing, i.e. the space formed by the volume between the inside of the top portion and the internal volume formed by the panels of the bottom portion and the intaglio surface. By removable attachment of the socket cap to the extraction site, a temporary seal is provided wherein an internal volume of the extraction site is maintained for further treatment while fluid and debris is prevented from entering the extraction site by the removable attachment to the tissue. In this configuration, the combination of the outer edge of the cap, engaging the circumference of the extraction site along an outer edge or an annular bottom portion thereof, together with the volume disposed by the bottom portion, creates a sealed inner volume within the extraction site such that the volume of the bottom portion extends into the extraction site and displaces at least a portion thereof to create a space. This, together with a sealing engagement of the top portion with the soft tissue around the extraction site, the socket cap provides both a fluid-tight seal about the periphery and a finite disposition of a volume within the extraction site. In another embodiment, the bottom portion will be made of solid construction with cylindrical walls of varying length, which extend into extraction sockets and an intaglio surface that may be flat, concave or convex in order to create a desired contour for the healed alveolar ridge. This will aid in fabrication of a future prosthesis which will replace the extracted tooth.

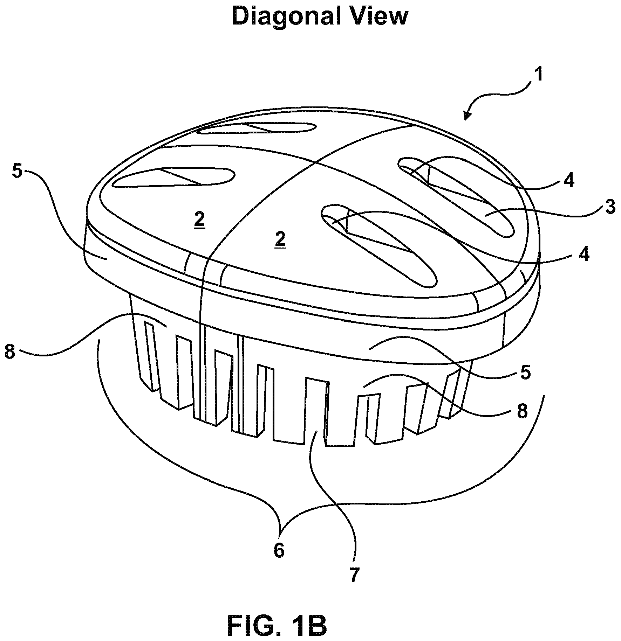

Turning to Figure 1A, a top view of the socket cap 1 shows an oval configuration having a plurality of grooves 3 formed in opposite sides of a dome shaped top portion 2 thereof. As shown in Figure 1B, the overall shape and configuration of the socket cap 1 may vary but is designed to provide a low profile inside the mouth when removably attached at the site. The exterior portion of the top 2 forms a sealing edge 5 to provide close engagement and a fluid seal with the soft tissue at the outer periphery of the extraction site when the cap 1 is affixed thereto. The dome shaped top portion 2 is preferably solid except for attachment means such as the grooves 3 and is manufactured with a thickness great enough to allow both the sealing function and the structural support for the downward bottom portion 6, as well as support for the grooves 3 and suture passages 4 as described below. In some embodiments, a tooth-shaped prosthesis may be attached to the top portion 2 to fill the space vacated by the extracted tooth or implant post.

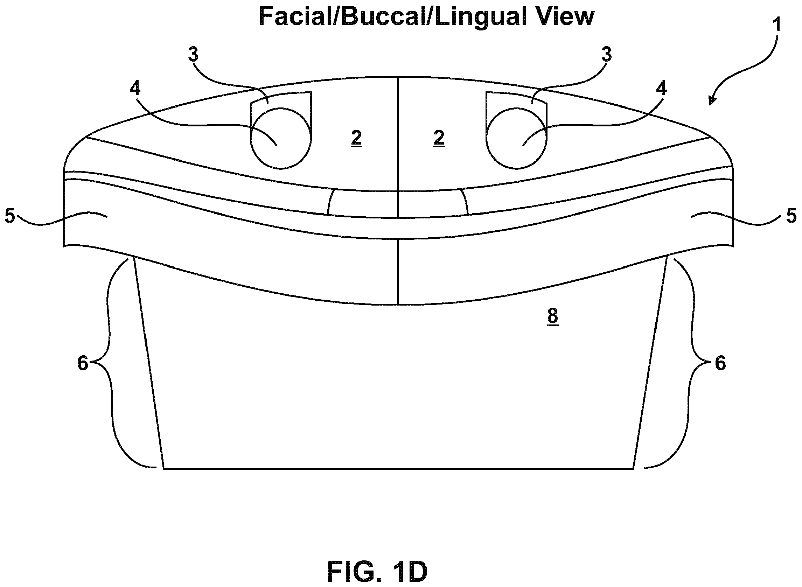

The top portion 2 is preferable solid across its entire surface except for the presence of the attachment means, i.e., grooves 3 and suture passages 4 or other attachment fixtures for screws, staples, clips or other mechanical expedients used to affix removable metal or synthetic materials to soft tissue, adjacent teeth or bone. As shown in Figure 1B and 1D, the grooves 3 lead to suture passages 4 that traverse the upper surface of the dome of the top portion 2 and form intact suture passages 4 that permit the surgeon to thread sutures passing through at least one passage 4 to secure the cap to tissue at the extraction site. The pairs of grooves 3 may also traverse the outer sealing edge 5 of the socket cap 1 or may be wholly formed in the surface of the top portion 2. In some embodiments, one or more perforations (not shown) traversing the height of the top portion 2 facilitate passage of a fixation screw to aid in the attachment of the cap to the socket. Alternative methods of securing the cap may be by application of adhesives, which may attach the cap to mucosa, bone, adjacent teeth or prosthesis. In some embodiment, there may be some features, which may aid in bonding the cap to adjacent teeth or prosthesis.

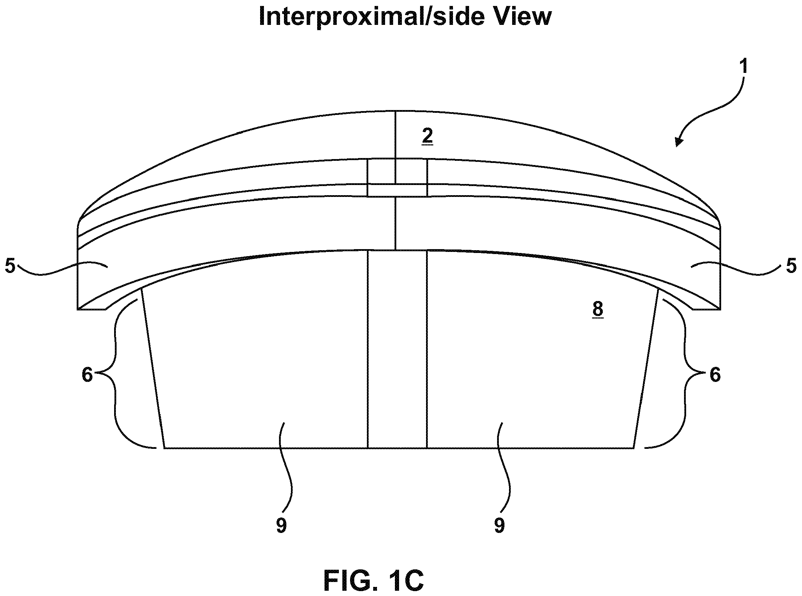

Referring again to Figure 1B, the sealing outer edge 5 of the top portion 2 may be rounded to provide close conforming engagement with the periphery of the extraction site and specifically for forming a fluid seal with the gingival margin. The housing 8 of the bottom portion 6 is preferably set inward from the edge 5 and are oriented to be internal to the outer circumference of the top portion 2 such that the overall area covered by the top portion 2 is greater than an area defined by the region of space created by the housing 8 at the bottom portion 6 and the internal area or volume thereby created within the cap 1 as defined by the structure of the combination of the top portion 2, the housing 8 and the base plate 9 (see Figure 1C). Accordingly, the area defined by the entire cap structure 1 preferably approximates the internal volume of the extraction site upon insertion when the sealing edge 5 contacts the tissue about the periphery thereof.

As will be appreciated from the diagonal view of Figure 1B, the housing 8 may be formed into downward extensions created by perforations 7 extending into the interior of the extraction site while the surface area provided by the top surface 2 and the outward edge 5 provide the sealing function against the soft tissue surrounding the extraction site. The number and orientation of perforations 7 are dictated only by practical considerations. As described in Figure 1A, either of the top portion 2 or the sealing edge 5 may have attachment means such as the grooves 3 with a dedicated suture passage 4 such that the surgeon may removably attach the device to tissue or
bone. Although in the embodiment of Figures 1A through 1E, the pairs of grooves 3 are linear and extend from one side of the rounded square cap top portion 2 to the other side, the position and orientation is not critical as long as the ability to provide a fixture where a suture secures the overall structure of the socket cap 1 to the surrounding soft tissue. For example, in some embodiment, there may be one or more openings through the top portion to facilitate passage of a fixation screw (not shown) to aid in the attachment of the cap to the socket.

Referring to Figure 1C, an interproximal side version of the socket cap of the present invention shows a solid structure for the bottom portion 6 integrally formed from the top portion 2 and defining an internal volume that defines the lateral dimension of the portion of the extraction site occupied by the cap device 1. The shape of the housing may be cylindrical, circular, or rounded square and has a circumferential surface abutting the sealing edge 5 about the periphery such that the exchange of blood and fluids across the gingival sulcus to the inner area of the extraction site is prevented.

The downward projections found in the bottom portion 6 features enough openness or perforations 7 such that the exchange of blood and fluids across the gingival sulcus to the inner area of the extension site is facilitated. The perforations 7 are preferably formed in the extensions 6 wherein the extensions are fabricated from conventional resorbable material such that any selected structure can dissolve during the healing process.

The general orientation of the bottom portion 6 includes a hollow cylindrical housing 8 comprised of panel structures 9 extending downward from the top portion 2 and preferably interior to the peripheral edge 5 such that a diameter of the housing 8 is less than the top portion 2 as defined by the diameter of the sealing edge 5. The height of the structures forming the bottom portion 6 is approximately equal to provide structural integrity and sealing about the periphery of the interior of the extraction site. The housing 8 may be substantially cylindrical or conformed to the shape of the top portion 2 of or the sealing edge 5. The housing 8 extends into the extraction socket to serve as a barrier between the mucosa and the socket lumen, which may be exposed as a result of dehiscence. Bone graft material inserted into the extraction site. The housing 8 prevents soft tissue from infiltrating the internal volume of the extraction site, which can contain bone graphed material.

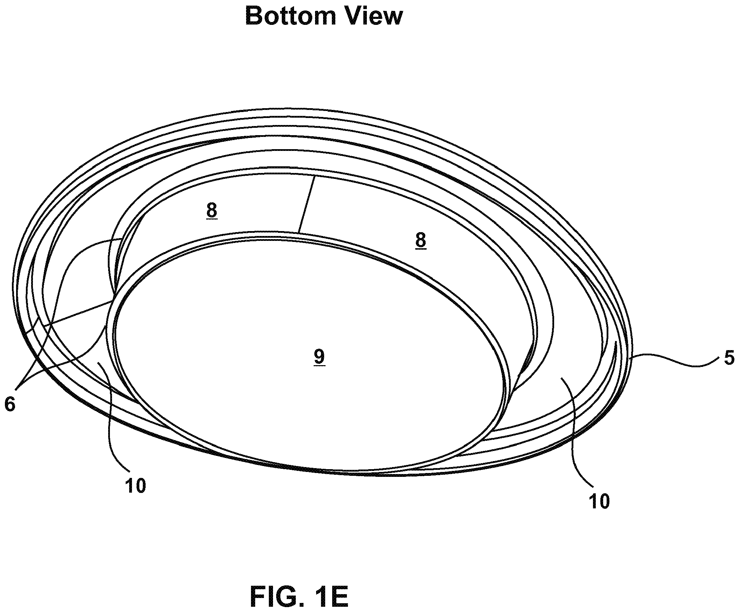

Figure 1D is a facial/buccal/labial view of the structures described with respect to Figure 1C above. As can be seen from the general orientation of the structures in Fig. 1D, the top portion 2 provides structural integrity and the placement of the attachment means, such as the grooves and grooves 3 and suture passages 4 formed in the top portion 2. The sealing edge 5 is oriented to prevent the passage of fluid or other materials from the oral cavity into the extraction site occupied by the volume displaced by the bottom portion 6. Where the housing 8 is intact and impregnable to fluid, the structure prevents fluid from passing from the space above the top portion 2 into the extraction site by virtue of the fluid impermeable barrier formed by sealing edge 5 and the soft tissue. As will be appreciated from the orientation of the sealing edge 5, the sealing edge 5 generally forms a circumferential barrier about the periphery of the top portion. The portion of the sealing edge 5 that contacts the soft tissue may be an annular orientation that is substantially planar or which has a curve as shown in Fig. 1D to accommodate sealing engagement between the sealing edge 5 and the soft tissue surrounding the extraction site. The sealing edge 5 is comprised of an annular seal that may be located at any portion of the sealing edge 5 including a lateral or bottom portion thereof. (See Figure 1E). The height of the bottom portion 6 is generally the same around the periphery of the structure such that the internal volume of the extraction site is defined by the volume of the bottom portion 6 of the cap 1. The entire cap device 1 may be symmetrical around a horizontal axis such that the surgeon need not be concerned with rotational placement of the cap 1.

Referring to Fig. 1E a bottom view of the socket cap device 1 shows the orientation of the bottom portion 6 comprises of the housing 8 and a base plate 9 that, by virtue of sealing engagement about the periphery with the housing 8, creates a fluid-tight internal volume within the housing 8 of the bottom portion 6. The sealing edge 5 may be comprised of an annular seal 10 located on the lower surface of the top portion 2 and having a flat surface that surrounds the bottom portion 6 and is oriented interior and circumferential to the outer portion of the sealing edge 5. The annular seal 10 is oriented between the sealing edge 5 and the bottom portion 6 and together create the fluid impregnable seal described above. The bottom plate 9 may be constructed in essentially any configuration that provides a seal with the housing 8 to create an intact, sealed inner volume in the lower portion 6. Because the embodiment comprising panels 9 are also sealed about the periphery of the annular ring seal 10 of the sealing edge 5, the entire structure is impregnable to fluid such that fluid or debris cannot pass from the oral cavity into the inner volume of the extraction site.

The entire structure of the socket cap 1 and any element thereof are fabricated from a variety of biocompatible materials including, but not limited to, polytetrafluoroethylene (PTFE), polypropylene, titanium, zirconia, polylactide, polylactic acid (PLA), polyglycolide, polyglycloic acid (PGA) or Polycaprolactone (PCL). In one embodiment, the top portion 2 of the cap device 1 comprising the top portion 2 and the sealing edge 5 are formed of non-degradable material, whereas the housing 8 and optimally the base plate 9 are fabricated from biodegradable material.

The overall structure of the socket cap may be fabricated from a variety of biocompatible resorbable or non-resorbable materials including, but not limited to, polytetrafluoroethylene (PTFE), polypropylene, collagen, hydroxyl apetite (HA), tricalcium phosphate, polyurethane, titanium, zirconia, polylactide, polylactic acid (PLA), polyglycolide, polyglycloic acid (PGA), Polycaprolactone (PCL), alginate, gelatin, hyaluronic acid or a combination of these material. In a preferred embodiment, the portion of the overall device comprising the top portion 2 and the sealing edge 5 are formed of non-degradable material, whereas the downward extensions and the housing 8 are fabricated from resorbable material that may be dissolved during healing of the extraction site. As noted above, the top portion 2 is typically a rounded square or rectangle and is preferably selected from sizes including 8mm by 9mm, 6mm x 6mm and 4mm by 4mm, although a variety of sizes are possible.. The thickness of the dome shaped portion is preferably thicker than 1/100^{th} of an inch and thicker than the housing 8, although the panels 9 when formed from
a resorbable material may necessarily be thicker and perforated, preferably ranging from 5 to 8/100ths of an inch.

Referring to Figures 2A-E, a second (non claimed) embodiment of the present invention is an extraction site socket cage that provides support for reconstruction of the extraction socket site, including both bone and soft tissue, particularly where portions of the structural aspect of the socket walls have been destroyed by infection, disease, trauma or damaged as a result of the surgical process to extract the tooth. The complete socket cage 10 assembly is comprised of a series of substantially horizontal beams with curved semi-circular structure, which are arranged in a manner analogous to ribs. The horizontal ribs are position with pre-defined space between them, which are connected with the aid of two vertical struts. The substantial horizontal orientation provides that the individual beams are roughly parallel and an individual beam does not contact an adjacent beam along the length thereof.

Referring to Figures 2A-2B, the vertical strut 11 provides the vertical length and points of attachment for the horizontal beams 15. The outer circumferential surface of each beam 15 engages the inner surfaces of the extraction site to prevent soft tissue growth towards the interior of the extraction site during healing. Ideally, the upper portion of the cage assembly has a larger diameter than the lower portion to accommodate the typical structure and dimensions of an extraction site. The outer curved circumferential surfaces 12 is preferably of substantially similar curvature or arc and together form a conical or generally cylindrical structure having one or more gaps 14 along the length thereof while also being structurally connected by the attachment to the vertical strut 11. A number of horizontal projections or beams 15 extending to form a roughly semi-circular profile to create curved walls or exterior circumferential surface. The horizontal beams 15 serve two purposes, to stabilize the cage into extraction socket walls and to provide spacers between the socket walls and the cage to allow for vascular supply to the socket walls.

As with the perforations in the embodiment described above, the curved outer surface 12 and optionally the vertical strut 11 have longitudinal spaces 14 that completely traverse the width of the beams 15 to provide for the passage of blood or other fluids within the extraction site. These spaces 14 also allow for exchange of vascular supply while preventing gingival soft tissues from proliferating into the socket. The socket cage 10 is preferably fabricated from the biocompatible and resorbable or non-resorbable materials listed above. In some embodiments, the extraction socket cap of Figures 1A-1E and the select cage at Figures 2A-2E may be fabricated as one combination device.

The sizing of the socket cage 10 is appropriate to accommodate the inner potions of typical extraction sites and include, but are not limited to, 11mm by 11mm, 8mm by 9mm, 6mm by 6mm and 4mm by 4mm. The longitudinal spaces 14 formed between the horizontal beams 15 of the cage 10 are roughly equivalent in shape between closely adjacent beams 14 to maintain the substantially equivalent curvature of adjustment horizontal beams 15 and all create the rib-like appearance of the overall structure.

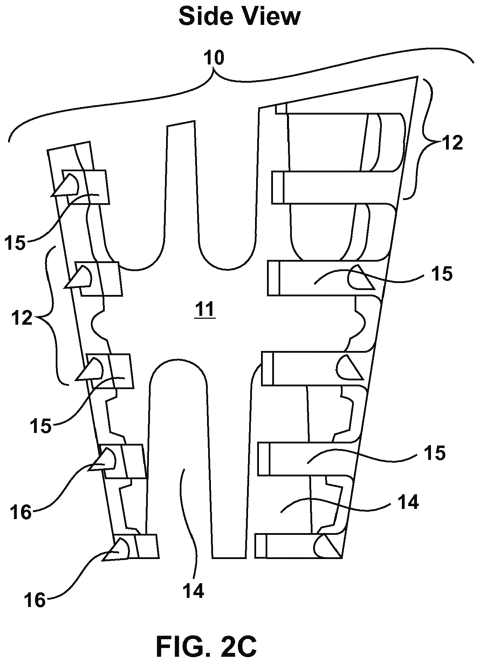

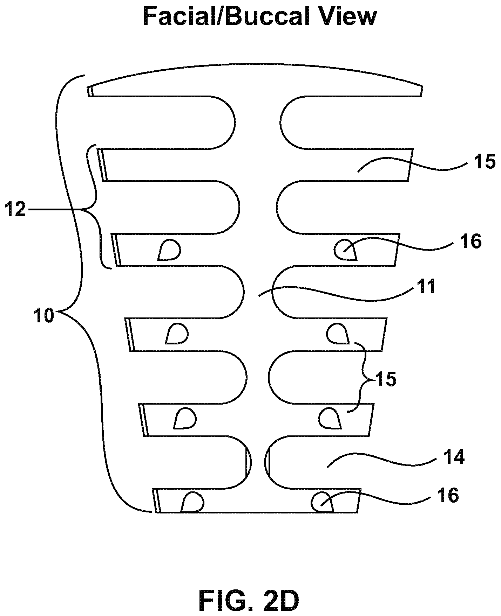

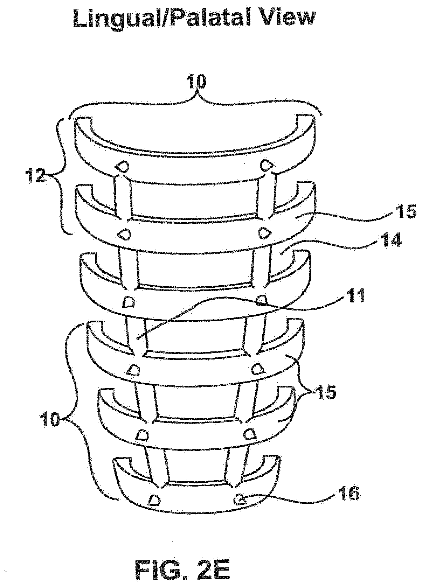

Referring to Figure 2C, the orientation of the horizontal beams 15 relative to each other and the longitudinal spaces 14 shows that the curvature or arc of the beams 15 is roughly equivalent on each opposing side of the semi-circular orientation. The vertical strut 11 shown in Figure 2C is optimal and in the absence thereof, the device assumes the configuration of Figures 2D or 2E where the horizontal beam members 15 are continuous and yield an arc approximating 180°, and greater than 90°, 100°, 110°, 120°, 130°, 140°, 150°, 160° or 170°. Fixation means are illustrated as sharpened points 16 extending radially from the outer circumferential surface 12 at the horizontal beams 15 but could be comprised of any structure or surface treatment to outer surface of the curved walls 12 such that horizontal, vertical, or rotational movement is impeded. The projections are radial, i.e., extending away from a central axis of the socket case 10 but need not be perpendicular thereto. Wing members (not shown) may privot away from the outer surface of the walls 12 and prevent movement in any direction.

Referring to Figure 2D, an embodiment is shown having a single vertical strut 11 disposed at a midpoint of each horizontal beam such that the length of each beam 15 extending away from the strut 11 is roughly equal. Fixation means 16 are located along the curved external surfaces 12 of a plurality of the beams 15. As in the embodiments described above, the diameter decreases from top to bottom to yield an inverted conical configuration. In practice, a pair of semi-circular socket cages may be placed within the extraction site to provide an annular support structure along the vertical height thereof. Figure 2E is an embodiment having a pair of vertical struts 11 oriented to be equally spaced from the end of each beam 15 to provide added structural integrity. The paired struts 11 are substantially parallel to each other except that the attachment points (or integral formation) to each beam 15 may form the tapered diameter at the cage 10 where a tapered conical configuration is desired. Preferably, the strut(s) 11, beams 15 and fixation means are integrally formed from a single piece of biocompatible material as described above.

The method of the present invention (not claimed) is to surgically place either or both an extraction socket cap 1 and an extraction cage 10 in an extraction site. Following extraction of a tooth or removal of a failed implant by conventional methods, the dentist or surgeon will prepare for an aseptic placement of the extraction cap 1 and/or cage 10 by examining the dimensions of the extraction site and determine whether any of the socket walls are defective. In case the socket walls have dehiscence, the cage will be applied. An appropriately sized cage will be selected and if necessary adjusted in dimensions. One method of adjustment may be to remove portions of the cage walls that may extend beyond the alveolar bone crest. If the cage is made of thermoplastic material, the cage will be heated to a temperature necessary to mold the cage to the geometry of the extraction socket. The cage is inserted with slight pressure inside the socket so that the sharp projects can engage the alveolar bone walls to stabilize the cage within the socket. The clinician may decide whether or not it is necessary to fill the socket with additional biomaterial. Such biomaterial may be osseoconductive to simply provide a matrix for bone growth or may include osseoinductive biomaterial to accelerate and enhance bone fill within the extraction socket. This method has significant advantages over current practices of treating extraction sockets. Currently, extraction sockets and grafts placed within the sockets are covered by resorbable or non-resorbable membranes. Such membranes are typically flat sheets of material ,which may be cut into a size suitable for the site. Membranes are typically either 1) positioned at the socket opening, while their margins are folded inward and are stabilized with sutures, or 2) positioned between periosteum and bone around the periphery of the extraction socket after a mucoperiosteal flap has been relected and then attached with suture. Disadvantages of current material and their application are 1) technical difficulty of adapting a flat membrane to the complex geometry of extraction socket opening, 2) generally poor seal made at the opening of sockets, 3) reduction of the bone crest after healing, as a results of utilizing flat membranes, 4) ineffectiveness of membranes in cases where extraction socket walls exhibit dehiscence, because membranes are flexible and their micromotion during healing favors fibrous tissue rather than bone formation and 5) potential for early loss of membranes, exposing the socket or the graft material contained within the socket, thereby compromising the outcomes. In contrast, the extraction socket caps and cages described herein offer significant advantages in each of the areas outlined, including, 1) extraction socket caps and cages are substantially easier to apply because of their prefabricated shape which can accommodate a variety of extraction socket morphologies, 2) extraction socket caps are designed for optimal sealing of the socket opening due to their conformation to typical socket gingival margins, 3) the dome shape of socket caps allow for bone graft to be placed and maintained at or above bone crest thereby minimizing the potential for alveolar ridge atrophy during the healing process, 4) socket cage due to its rigid construction can maintain and reconstruct the lost socket walls during the healing process and 5) the socket caps feature improved methods for stabilization, ensuring their stability during the entire healing period.

The placement of extraction socket cap 1 is initiated by measuring the dimensions of the extraction socket opening to select an appropriate size cap. The extraction socket may be prepared as deemed appropriate by the clinician. The extractions socket cap 1 may be adjusted to better fit the dimensions of the socket. This may entail reducing some of the peripheral rim or projections of the cap. If the cap is made of thermoplastic material, the cap will be heated to a temperature necessary to mold the cap to the geometry of the extraction socket. The engaging edge forms an effective fluid seal around the periphery of the site at the gingival sulcus. Next, the cap is fixated to the extraction socket. Under one embodiment, the socket cap is attached to extraction socket by means of suture. In this case, sutures will engage soft tissues surrounding extraction socket and passed through the suture channels incorporated into the design of the cap. Another embodiment may have sutures that are already incorporated and contiguous with the cap for ease of fixation. Another embodiment may utilize other means to anchor the cap to the extraction socket. This may entail utilization of fixation screws, hooks, adhesives or other mechanical, physical or chemical means to attach the cap to the extraction socket.

## Claims

1. A dental socket cap for placement in an extraction site comprising:
a top portion (2) having attachment means and a sealing edge (5) about a periphery thereof for creating a fluid seal with soft tissue, wherein the attachment means is a groove (3) operably connected to a suture passage (4) traversing the top portion (2); and
a bottom portion (6) having a housing (8) defining an internal volume created by a combination of the top portion (2) and the bottom portion (6),
wherein the sealing edge (5) has a larger diameter than the bottom portion (6).

2. The dental socket cap of claim 1, wherein the top portion (2) is dome-shaped.

3. The dental socket cap of claim 2, wherein attachment means are disposed within the dome shaped portion (2).

4. The dental socket cap of claim 1, wherein the bottom portion (6) is further comprised of a base plate (9).

5. The dental socket cap of claim 4, wherein the base plate (9) is sealed about a periphery thereof to the housing (8) of the bottom portion (6).

6. The dental socket cap of claim 1, wherein the sealing edge (5) is further comprised of an annular seal (10) that surrounds the housing (8).

7. The dental socket cap of claim 6, wherein the annular seal (10) is oriented on a lower surface of the top portion (2).

## Patentansprüche

1. Extraktionsalveolenverschluss zur Platzierung in einer Extraktionsstelle, umfassend:
einen oberen Teil (2) mit Befestigungsmitteln und einem Dichtrand (5) um einen Umfang davon zum Erzeugen einer Fluiddichtung gegenüber Weichgewebe, wobei die Befestigungsmittel aus einer Vertiefung (3) bestehen, die mit einem Nahtdurchgang (4) wirkverbunden ist, der durch den oberen Teil (2) führt; und
einen unteren Teil (6) mit einem Gehäuse (8), der ein Innenvolumen definiert, das durch eine Kombination des oberen Teils (2) und des unteren Teils (6) erzeugt wird,
wobei der Dichtungsrand (5) einen größeren Durchmesser aufweist als der untere Teil (6).

2. Extraktionsalveolenverschluss nach Anspruch 1, wobei der obere Teil (2) gewölbt ist.

3. Extraktionsalveolenverschluss nach Anspruch 2, wobei Befestigungsmittel innerhalb des gewölbten Teils (2) angeordnet sind.

4. Extraktionsalveolenverschluss nach Anspruch 1, wobei der untere Teil (6) ferner aus einer Basisplatte (9) besteht.

5. Extraktionsalveolenverschluss nach Anspruch 4, wobei die Basisplatte (9) an einem Umfang davon mit dem Gehäuse (8) des unteren Teils (6) abgedichtet ist.

6. Extraktionsalveolenverschluss nach Anspruch 1, wobei der Dichtungsrand (5) ferner aus einer ringförmigen Dichtung (10) besteht, die das Gehäuse (8) umgibt.

7. Extraktionsalveolenverschluss nach Anspruch 6, wobei die ringförmige Dichtung (10) auf einer unteren Fläche des oberen Teils (2) ausgerichtet ist.

## Revendications

1. Capuchon de douille dentaire destiné à être placé dans un site d'extraction, comprenant :
une partie supérieure (2) comportant des moyens de fixation et un bord d'étanchéité (5) autour d'une périphérie de celle-ci pour créer un joint d'étanchéité aux fluides avec un tissu mou, dans lequel les moyens de fixation sont une rainure (3) reliée fonctionnellement à un passage de suture (4) traversant la partie supérieure (2) ; et
une partie inférieure (6) ayant un boîtier (8) définissant un volume interne créé par une combinaison de la partie supérieure (2) et de la partie inférieure (6),
dans lequel le bord d'étanchéité (5) a un diamètre plus grand que la partie inférieure (6).

2. Capuchon de douille dentaire selon la revendication 1, dans lequel la partie supérieure (2) est en forme de dôme.

3. Capuchon de douille dentaire selon la revendication 2, dans lequel des moyens de fixation sont disposés à l'intérieur de la partie en forme de dôme (2).

4. Capuchon de douille dentaire selon la revendication 1, dans lequel la partie inférieure (6) est en outre constituée d'une plaque de base (9).

5. Capuchon de douille dentaire selon la revendication 4, dans lequel la plaque de base (9) est scellée autour d'une périphérie de celle-ci au boîtier (8) de la partie inférieure (6).

6. Capuchon de douille dentaire selon la revendication 1, dans lequel le bord d'étanchéité (5) est en outre constitué d'un joint d'étanchéité annulaire (10) qui entoure le boîtier (8).

7. Capuchon de douille dentaire selon la revendication 6, dans lequel le joint d'étanchéité annulaire (10) est orienté sur une surface inférieure de la partie supérieure (2).
